# EUROPEAN PATENT SPECIFICATION

(11) **EP 4 162 977 B1**
(45) Date of publication and mention of the grant of the patent: **19.03.2025**
(21) Application number: 22772396.2
(22) Date of filing: 02.05.2022
(51) Int. Cl.: A61N 1/40, A61N 1/06, A61N 1/08, A61N 1/04, A61N 1/32, A61N 1/36, A61H 9/00

(54) **BODY CARE DEVICE**
HAUTBEHANDLUNGSVORRICHTUNG
DISPOSITIF DE SOINS CORPORELS

(30) Priority: 26.08.2021 KR 20210113409
(43) Date of publication of application: 12.04.2023
(73) Proprietor: APR CO., LTD., Seoul 05551 (KR)
(72) Inventor: LEE, Jeong Ho, Seoul 07764 (KR)
(74) Representative: Ruttensperger Lachnit Trossin Gomoll
(86) International application number: PCT/KR2022/006281
(87) International publication number: WO 2023/027289

(56) References cited:
- WO-A1-2017/099386
- KR-B1- 101 292 562
- KR-B1- 101 540 832
- KR-B1- 101 859 195
- KR-B1- 102 129 165
- KR-U- 20090 011 617
- US-A1- 2012 197 242
- US-B2- 10 918 859

## Description

### TECHNICAL FIELD

The disclosure relates to a skin management device, and more particularly, to a skin management device which recognizes whether the user's skin has been sucked, and when the user's skin has been sucked, applies a high frequency to the sucked skin, thereby reducing power consumption and improving a skin massage effect.

### BACKGROUND ART

In order to prevent skin aging, various instruments for massaging the skin have been developed. Recently, various apparatuses for providing a massage effect by using a high frequency or a low frequency have been released.

For a device that provides a massage effect by using a high frequency or a low frequency, skin is sucked by a suction cup, and a high frequency or a low frequency is provided to the skin sucked into the suction cup.

However, as the high frequency or low frequency is provided without recognizing whether the user's skin is appropriately sucked into the suction cup, unnecessary power may be wasted. In addition, the skin massage effect may also be degraded.

A skin management device according to the preamble of claim 1 is known from US 2012/017242 A1. In this known skin management device, a skin protrusion sensor provides a signal indicating that the skin to be treated fills a cavity into which the skin is sucked due to a negative pressure generated by a negative pressure source. Based on this signal, a RF generator is controlled for supplying electric energy to electrodes only in a condition in which the skin to be treated fills the cavity.

### DESCRIPTION OF EMBODIMENTS

### TECHNICAL PROBLEM

It is the object of the present invention to provide a skin management device which is able of sucking a user's skin in a desired extent, and when the user's skin has been sucked in the desired extent, applies a high frequency to the sucked skin, thereby reducing power consumption and improving a skin massage effect.

### SOLUTION TO PROBLEM

According to the present invention, this object is achieved by a skin management device according to claim 1. This skin management device includes a main body portion having a suction cup, an electrode tip provided on an inner circumferential surface of the suction cup, a high frequency generation portion configured to apply a high frequency to the electrode tip, a suction pump configured to provide suction power to the suction cup, a contact sensor provided on the inner circumferential surface of the suction cup and disposed around the electrode tip, and a controller configured to control operations of the high frequency generation portion and the suction pump, wherein the suction pump operates to suck the user's skin into the suction cup, and when the user's skin contacts the contact sensor, the contact sensor transmits a contact signal to the controller, and the controller, when receiving the contact signal, recognizes suction of the user's skin and operates the high frequency generation portion to apply a high frequency to the user's skin through the electrode tip.

In the skin management device according to the present invention, the contact sensor includes a first contact sensor and a second contact sensor. The first contact sensor is disposed at a position lower than the second contact sensor. The first contact sensor generates a first contact signal, and the second contact sensor generates a second contact signal. The controller, when receiving the first contact signal, operates the high frequency generation portion, and when receiving the second contact signal, controls operation of the suction pump.

In the skin management device according to an embodiment of the disclosure, when the contact signal is removed, the controller may stop the operation of the high frequency generation portion.

In the skin management device according to an embodiment of the disclosure, the electrode tip may be disposed on an inner circumferential surface of the main body portion to protrude inwardly.

In the skin management device according to an embodiment of the disclosure, the electrode tip may include a positive tip and a negative tip, and the positive tip and the negative tip may be disposed on an inner circumferential surface of the main body portion at positions facing each other.

In the skin management device according to an embodiment of the disclosure, the contact sensor may be disposed at a position parallel to or lower than the electrode tip.

In the skin management device according to an embodiment of the disclosure, the controller, when receiving the second contact signal, may stop operation of the suction pump, or reduces suction power of the suction pump.

The skin management device according to an embodiment of the disclosure may further include a temperature sensor disposed around the electrode tip, wherein the controller is further configured to provide a notification or control operation of the high frequency generation portion, according to a temperature sensed by the temperature sensor.

### ADVANTAGEOUS EFFECTS OF DISCLOSURE

In the skin management device according to the disclosure, whether the user's skin has been sucked is recognized, and when the user's skin has been sucked, a high frequency is applied to the sucked skin, power consumption may be saved and a skin massage effect may be improved.

In addition, in the skin management device according to the disclosure, when it is determined that the user's skin is excessively sucked, or the temperature is too high, the operations of the suction pump and the high frequency generation portion are controlled so that damage to the user's skin may be prevented and safety accidents may be prevented from occurring.

### BRIEF DESCRIPTION OF DRAWINGS

FIGS. 1 to 3 illustrate the appearance of a skin management device according to an embodiment of the disclosure.
FIG. 4 illustrates the inner structure of the skin management device according to an embodiment of the disclosure.
FIG. 5 illustrates the inner structure of a skin management device according to an embodiment of the disclosure.
FIGS. 6 and 7 illustrate the use and operation of a skin management device according to an embodiment of the disclosure.

### MODE OF DISCLOSURE

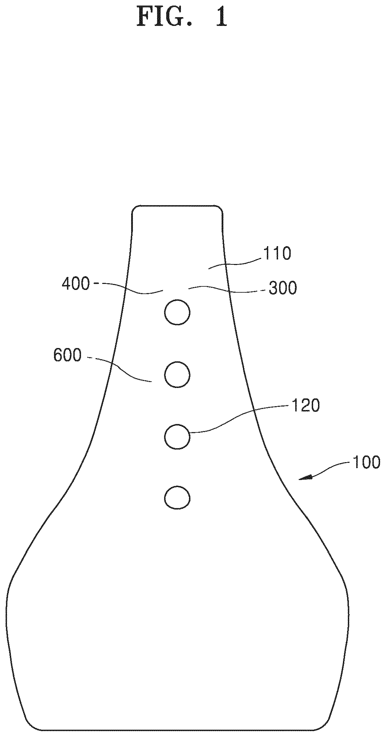

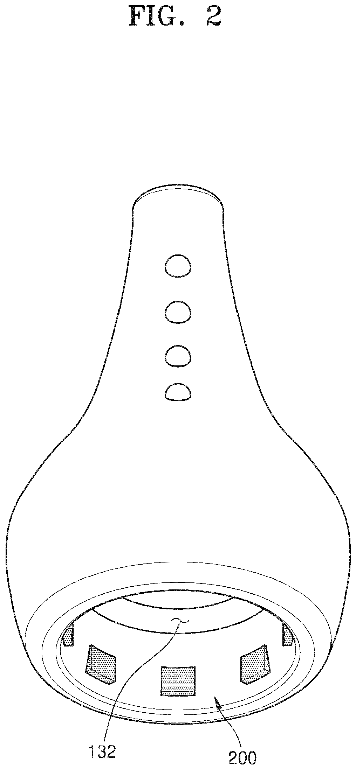

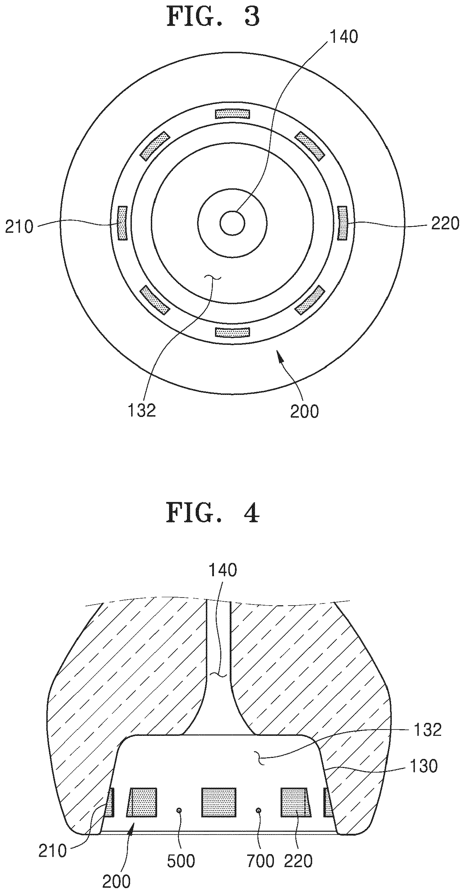

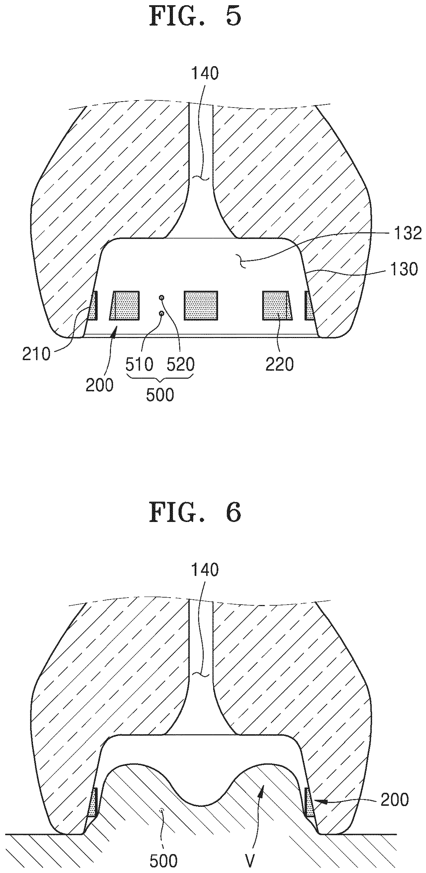

Hereinafter, the disclosure will be described more fully with reference to the accompanying drawings
FIGS. 1 to 3 illustrate the appearance of a skin management device according to an embodiment of the disclosure. FIG. 4 illustrates the inner structure of the skin management device according to an embodiment of the disclosure. FIG. 5 illustrates the inner structure of the skin management device according to an embodiment of the disclosure.

The skin management device according to an embodiment of the disclosure includes a main body portion 100 having a suction cup 130, an electrode tip 200 provided on the inner circumferential surface of the suction cup 130, a high frequency generation portion 300 for applying a high frequency to the electrode tip 200, a suction pump 400 for providing suction power to the suction cup 130, a contact sensor 500 provided on the inner circumferential surface of the suction cup 130 and disposed around the electrode tip 200, and a controller 600 for controlling the operations of the high frequency generation portion 300 and the suction pump 400. When the suction pump 400 operates to suck the user's skin into the main body portion 100 and the user's skin contacts the contact sensor 500, the controller 600 recognizes that the user's skin is sucked and operates the high frequency generation portion 300 to apply a high frequency to the user's skin through the electrode tip 200.

The main body portion 100 forms the appearance of the skin management device.

The main body portion 100 may have a shape and structure that enables a user to conveniently grip and carry a skin management device according to the disclosure. However, the appearance of the main body portion 100 is not necessarily limited to the shape illustrated in the drawings. A grip portion 110 for gripping by a user may be provided in an upper portion of the main body portion 100. The grip portion 110, which has an appropriate structure and shape for a user to grip, may be a portion having a relatively small outer diameter compared with other portions, as illustrated in the drawings, or a constricted portion or a portion with a handle.

A control portion 120 for control by a user may be provided on the outer surface of the main body portion 100. The control portion 120 may include buttons or a display for outputting input buttons. A user's input signal may be input through the control portion 120. Although not illustrated, an output portion for checking an operating state may be provided on the outer surface of the main body portion 100.

The suction cup 130 may be provided in a lower portion of the main body portion 100. The suction cup 130 has a cup shape, and the suction cup 130 has a suction space 132 open in a downward direction. A suction hole 140 opened in an upward direction may be provided in the upper portion of the suction cup 130. The suction hole 140 communicates with the suction pump 400 described below.

The high frequency generation portion 300, the suction pump 400, and the controller 600 described below may be included in the main body portion 100. In addition, various power portions for providing power may be mounted in the main body portion 100.

The electrode tip 200 is provided on the inner circumferential surface of the suction cup 130.

The electrode tip 200 may include a plurality of electrode tips. For example, although FIG. 3 illustrates that there are eight electrode tips as the electrode tip 200, the disclosure is not limited thereto. The electrode tips 200 may be disposed at intervals along the perimeter of the inner circumferential surface of the suction cup 130.

Each of the electrode tips 200 may use a single metal or alloy having conductivity as a base substrate. According to an embodiment, each of the electrode tips 200 may include a component that can deliver a stable high frequency output to a fat layer deep in the skin without any trouble due to contact with the skin, and increase heat generation due to stimulation of the fat layer and decomposition efficiency.

According to an embodiment of the disclosure, the electrode tips 200 may be disposed on the inner circumferential surface of the main body portion 100, and may protrude toward the inside of the main body portion 100. Accordingly, each of the electrode tips 200 may effectively transmit a high frequency output to the user's skin sucked in the main body portion 100.

According to an embodiment of the disclosure, each of the electrode tips 200 may include a positive tip 210 and a negative tip 220. According to an embodiment, the positive tip 210 and the negative tip 220 may be disposed at positions facing each other on the inner circumferential surface of the main body portion 100.

The high frequency generation portion 300 may be mounted in the main body portion 100. The high frequency generation portion 300 is a member that outputs high frequency energy and radiates the high frequency energy through the electrode tips 200. The high frequency energy may refer to energy having a certain frequency to generate an effect in skin management and improvement. As an example, the high frequency energy may be a "radio frequency (RF)." The high frequency generation portion 300 may be any device capable of generating the high frequency energy, and a detailed structure thereof is not particularly limited. In other words, the high frequency generation portion 300 may have a structure that can be understood by a person skilled in the art to which the disclosure pertains can understand.

The suction pump 400 is a member that communicates with the suction hole 140 and provides suction power to the suction cup 130 through the suction hole 140. The specific structure and type of the suction pump 400 are not limited. The suction pump 400 may be, for example, a vacuum pump. According to an embodiment, the suction power provided by the suction pump 400 may vary.

The contact sensor 500 generates a signal when contacting the user's skin. According to an embodiment, the contact sensor 500 may be disposed on the inner circumferential surface of the suction cup 130.

According to an embodiment, the contact sensor 500 may be disposed at a position parallel to or lower than the electrode tip 200, in a vertical direction of the main body portion 100.

As shown in the embodiment, as the contact sensor 500 is disposed at a position parallel to or lower than the electrode tip 200, the contact sensor 500 may easily recognize the user's skin sucked into the main body portion 100. In other words, when the user's skin has been sucked into the suction cup 130, as the user's skin comes into contact with the contact sensor 500, the contact sensor 500 may recognize that the user's skin is sucked and positioned in a contact state with the electrode tip 200.

According to the present invention, the contact sensor 500 includes a plurality of contact sensors. The contact sensor 500 includes a first contact sensor 510 and a second contact sensor 520, and the arrangement height of the first contact sensor 510 and the arrangement height of the second contact sensor 520 is different from each other. In other words, the first contact sensor 510 is at a relatively low position and the second contact sensor 520 is at a relatively higher position than the first contact sensor 510.

The first contact sensor 510 may be positioned, as described above, at a position parallel to or lower than the electrode tip 200. In addition, the second contact sensor 520 may be positioned at a position higher than the electrode tip 200.

The controller 600 may be mounted in the main body portion 100. The controller 600 may be a CPU. The controller 600 may generate an output signal in response to signals provided from the control portion 120, the contact sensor 500, or a temperature sensor 700 described below. The controller 600 may control the operations of the high frequency generation portion 300 and the suction pump 400 by using the output signal.

The skin management device according to an embodiment of the disclosure may further include the temperature sensor 700 around the electrode tips 200. In addition, the controller 600 may provide a notification, control the operation of the suction pump 400, or control the operation of the high frequency generation portion 300, according to a temperature sensed by the temperature sensor 700.

As an example, when the temperature sensed by the temperature sensor 700 is greater than or equal to a reference temperature, the operation of the suction pump 400 may be stopped, or the suction power of the suction pump 400 may be reduced. In another example, when the temperature sensed by the temperature sensor 700 is greater than or equal to a reference temperature, the operation of the high frequency generation portion 300 may be stopped, or the output generated from the high frequency generation portion 300 may be reduced.

As described above, as the temperature sensor 700 is provided, and the controller 600 controls the operations of the suction pump 400 and the high frequency generation portion 300 in response to the signal output from the temperature sensor 700, safety accidents may be prevented from occurring.

For example, when the high frequency energy applied to the user's skin increases too high, or is provided for a long time, the temperature of the user's skin may be increased too high. In this case, a user may be injured, such as burns. According to an embodiment, in this case, as the controller 600 appropriately controls the operation of the high frequency generation portion 300, the user may be prevented from being injured by burns and the like.

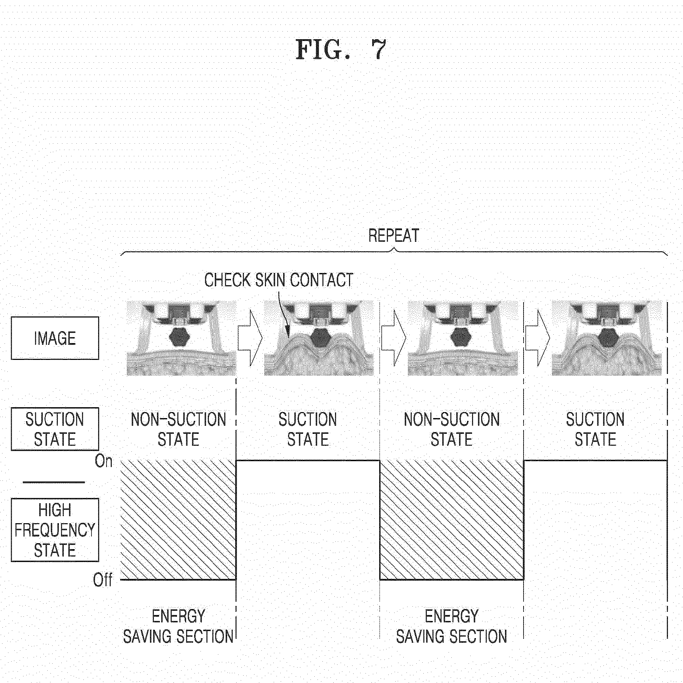

FIGS. 6 and 7 illustrate the use and operation of a skin management device according to an embodiment of the disclosure.

In the following description, the operation of the skin management device according to the embodiment of the disclosure is described.

When the suction cup 130 of the main body portion 100 is brought into close contact with the user's skin and the suction pump 400 is operated by manipulating the control portion 120, the user's skin is sucked into the suction space 132 of the suction cup 130. In this state, as the user's skin is in contact with the contact sensor 500 in the suction cup 130, the contact sensor 500 provides the controller 600 with a contact signal. The contact signal is a signal indicating that the user's skin is sucked into the suction cup 130 and reaches a certain position. The controller 600 that received the contact signal recognizes that the user's skin has been sucked into the suction space 132, and operates the high frequency generation portion 300 to provide an ON output signal that provides high frequency energy.

When the suction by the suction pump 400 is stopped or the user's skin escapes from the suction cup 130, the contact sensor 500 provides the controller 600 with an escape signal. The escape signal is a signal indicating that the user's skin has escaped from the suction cup 130. The controller 600 that received the escape signal recognizes that the user's skin has escaped from the suction space 132, and stops the operation of the high frequency generation portion 300 to provide an OFF output signal that stops the provision of the high frequency energy.

The escape signal may mean, for example, the skin contact signal is removed because the user's skin is not in contact with the contact sensor 500. In other words, when sensing that the user's skin has escaped from the contact sensor 500, the contact sensor 500 may generate an escape signal, or stops an operating signal, so that the controller 600 may stop the operation of the high frequency generation portion 300.

FIG. 7 illustrates the operation of the skin management device according to an embodiment of the disclosure. As illustrated in FIG. 7, in a non-suction state in which the user's skin is not sucked into the suction cup 130, the high frequency generation portion 300 is in an OFF state. In a suction state in which the user's skin is sucked into the suction cup 130, the high frequency generation portion 300 is in an ON state. As in an embodiment, as the ON/OFF of the high frequency generation portion 300 is determined depending on the suction of the user's skin, consumption power may be saved, and energy efficiency may be increased.

As the contact sensor 500 includes the first contact sensor 510 and the second contact sensor 520, the controller 600, when receiving a contact signal from the first contact sensor 510, may provide the high frequency generation portion 300 with an ON output signal, and the controller 600, when receiving a contact signal from the second contact sensor 520, may provide an OFF output signal that turns the suction pump 400 off.

In other words, in the skin management device according to the present invention, when the suction power of the suction pump 400 is so strong that the user's skin is sucked too much and then the user's skin is sucked to a position where the second contact sensor 520 is disposed, the suction pump 400 is turned off so that the user's skin may be prevented from being damaged. Furthermore, in this state, the suction pump 400 may be turned off until the contact signal from the second contact sensor 520 is removed. According to another embodiment, it is possible to generate a signal that decreases the suction power of the suction pump 400, until the contact signal from the second contact sensor 520 is removed.

Unlike the above-described embodiments, it is possible that the contact sensor 500 may have a different structure. In other words, the contact sensor 500 does not necessarily provide a signal only through contact/non-contact of skin, but is possible to generate a signal when skin reaches a certain position or height. In other words, the contact sensor 500 may mean any sensors, and can be understood as the term "sensor unit." As an example, the sensor unit in lieu of the contact sensor may be, for example, an optical sensor, and the optical sensor may irradiate light to a certain height, and when the user's skin reaches the height, the sensor unit may provide a signal to the controller 600. The above descriptions may be applied to the location and operation of the sensor unit.

In the skin management device according to the disclosure, whether the user's skin has been sucked is recognized, and when the user's skin has been sucked, a high frequency is applied to the sucked skin, power consumption may be saved and a skin massage effect may be improved.

In addition, in the skin management device according to the disclosure, when it is determined that the user's skin is excessively sucked, or the temperature is too high, the operations of the suction pump 400 and the high frequency generation portion 300 are controlled so that damage to the user's skin may be prevented and safety accidents may be prevented from occurring.

## Claims

1. A skin management device comprising:
a main body portion (100) having a suction cup (130);
an electrode tip (200) provided on an inner circumferential surface of the suction cup (130);
a high frequency generation portion (300) configured to apply a high frequency to the electrode tip (200);
a suction pump (400) configured to provide suction power to the suction cup (130);
a contact sensor (500) provided on the inner circumferential surface of the suction cup (130) and disposed around the electrode tip (200); and
a controller (600) configured to control operations of the high frequency generation portion (300) and the suction pump (400),
wherein the suction pump (400) operates to suck a user's skin into the suction cup (130), and when the user's skin contacts the contact sensor (500), the contact sensor (500) transmits a contact signal to the controller (600), and
the controller (600), when receiving the contact signal, recognizes suction of the user's skin and operates the high frequency generation portion (300) to apply a high frequency to the user's skin through the electrode tip (200),
**characterized in that**:
the contact sensor (500) comprises a first contact sensor (510) and a second contact sensor (520),
the first contact sensor (510) is disposed at a position lower than the second contact sensor (520),
the first contact sensor (510) generates a first contact signal,
the second contact sensor (520) generates a second contact signal, and
the controller (600), when receiving the first contact signal, operates the high frequency generation portion (300), and when receiving the second contact signal, controls operation of the suction pump (400).

2. The skin management device of claim 1, wherein, when the contact signal is removed, the controller (600) stops the operation of the high frequency generation portion (300).

3. The skin management device of claim 1, wherein the electrode tip (200) is disposed on an inner circumferential surface of the main body portion (100) to protrude inwardly.

4. The skin management device of claim 1, wherein the electrode tip (200) comprises a positive tip (210) and a negative tip (220), and
the positive tip (210) and the negative tip (220) are disposed on an inner circumferential surface of the main body portion (110) at positions facing each other.

5. The skin management device of claim 1, wherein the contact sensor (500) is disposed at a position parallel to or lower than the electrode tip (200).

6. The skin management device of claim 1, wherein the controller (600), when receiving the second contact signal, stops operation of the suction pump (400), or reduces suction power of the suction pump (400).

7. The skin management device of claim 1, further comprising a temperature sensor (700) disposed around the electrode tip,
wherein the controller (600) is further configured to provide a notification or control operation of the high frequency generation portion, according to a temperature sensed by the temperature sensor (700).

## Patentansprüche

1. Hautbehandlungsvorrichtung, umfassend:
einen Hauptkörperabschnitt (100) mit einem Saugnapf (130);
eine Elektrodenspitze (200), die auf einer inneren Umfangsfläche des Saugnapfs (130) vorgesehen ist;
einen Hochfrequenzerzeugungsabschnitt (300), der dazu ausgebildet ist, eine Hochfrequenz an die Elektrodenspitze (200) anzulegen;
eine Saugpumpe (400), die dazu ausgebildet ist, den Saugnapf (130) mit Saugkraft zu versorgen;
einen Kontaktsensor (500), der auf der inneren Umfangsfläche des Saugnapfes (130) vorgesehen und um die Elektrodenspitze (200) herum angeordnet ist; und
eine Steuerung (600), die dazu ausgebildet ist, den Betrieb des Hochfrequenzerzeugungsabschnitts (300) und der Saugpumpe (400) zu steuern, wobei die Saugpumpe (400) so arbeitet, dass sie die Haut eines Benutzers in den Saugnapf (130) saugt, und wenn die Haut des Benutzers den Kontaktsensor (500) berührt, der Kontaktsensor (500) ein Kontaktsignal an die Steuerung (600) sendet, und
die Steuerung (600), wenn sie das Kontaktsignal empfängt, das Ansaugen der Haut des Benutzers erkennt und den Hochfrequenzerzeugungsabschnitt (300) betreibt, um eine Hochfrequenz über die Elektrodenspitze (200) an die Haut des Benutzers anzulegen,
**dadurch gekennzeichnet, dass**:
der Kontaktsensor (500) einen ersten Kontaktsensor (510) und einen zweiten Kontaktsensor (520) umfasst,
wobei der erste Kontaktsensor (510) an einer Position angeordnet ist, die niedriger ist als die des zweiten Kontaktsensors (520),
der erste Kontaktsensor (510) ein erstes Kontaktsignal erzeugt,
der zweite Kontaktsensor (520) ein zweites Kontaktsignal erzeugt und
die Steuerung (600) bei Empfang des ersten Kontaktsignals den Hochfrequenzerzeugungsabschnitt (300) betreibt und bei Empfang des zweiten Kontaktsignals den Betrieb der Saugpumpe (400) steuert.

2. Hautbehandlungsvorrichtung nach Anspruch 1, wobei die Steuerung (600) den Betrieb des Hochfrequenzerzeugungsabschnitts (300) stoppt, wenn das Kontaktsignal entfernt wird.

3. Hautbehandlungsvorrichtung nach Anspruch 1, wobei die Elektrodenspitze (200) auf einer inneren Umfangsfläche des Hauptkörperabschnitts (100) angeordnet ist, um nach innen vorzustehen.

4. Hautbehandlungsvorrichtung nach Anspruch 1, wobei die Elektrodenspitze (200) eine positive Spitze (210) und eine negative Spitze (220) umfasst, und
die positive Spitze (210) und die negative Spitze (220) an einander zugewandten Positionen an einer Innenumfangsfläche des Hauptkörperabschnitts (110) angeordnet sind.

5. Hautbehandlungsvorrichtung nach Anspruch 1, wobei der Kontaktsensor (500) an einer Position parallel zu oder unterhalb der Elektrodenspitze (200) angeordnet ist.

6. Hautbehandlungsvorrichtung nach Anspruch 1, wobei die Steuerung (600) bei Empfang des zweiten Kontaktsignals den Betrieb der Saugpumpe (400) stoppt oder die Saugleistung der Saugpumpe (400) reduziert.

7. Hautbehandlungsvorrichtung nach Anspruch 1, welche ferner einen Temperatursensor (700) umfasst, der um die Elektrodenspitze herum angeordnet ist,
wobei die Steuerung (600) ferner dazu ausgebildet ist, eine Benachrichtigung oder einen Steuerungsvorgang des Hochfrequenzerzeugungsabschnitts gemäß einer von dem Temperatursensor (700) erfassten Temperatur bereitzustellen.

## Revendications

1. Un dispositif de traitement de la peau comprenant :
une partie de corps principal (100) comportant une ventouse (130) ;
une pointe d'électrode (200) située sur une surface circonférentielle interne de la ventouse (130) ;
une partie de génération de haute fréquence (300) configurée pour appliquer une haute fréquence à la pointe d'électrode (200) ;
une pompe d'aspiration (400) configurée pour fournir une puissance d'aspiration à la ventouse (130) ;
un capteur de contact (500) situé sur la surface circonférentielle interne de la ventouse (130) et disposé autour de la pointe d'électrode (200) ; et
une commande (600) configurée pour commander les opérations de la partie de génération de haute fréquence (300) et de la pompe d'aspiration (400),
dans lequel la pompe d'aspiration (400) fonctionne pour aspirer la peau d'un utilisateur dans la ventouse (130), et lorsque la peau de l'utilisateur entre en contact avec le capteur de contact (500), le capteur de contact (500) transmet un signal de contact à la commande (600), et
la commande (600), lorsqu'elle reçoit le signal de contact, reconnaît l'aspiration de la peau de l'utilisateur et fait fonctionner la partie de génération de haute fréquence (300) pour appliquer une haute fréquence à la peau de l'utilisateur par l'intermédiaire de la pointe d'électrode (200),
**caractérisé en ce que** :
le capteur de contact (500) comprend un premier capteur de contact (510) et un second capteur de contact (520),
le premier capteur de contact (510) est disposé à une position inférieure au second capteur de contact (520),
le premier capteur de contact (510) génère un premier signal de contact,
le second capteur de contact (520) génère un second signal de contact, et
la commande (600), lorsqu'elle reçoit le premier signal de contact, fait fonctionner la partie de génération de haute fréquence (300), et lorsqu'il reçoit le second signal de contact, commande le fonctionnement de la pompe d'aspiration (400).

2. Le dispositif de traitement de la peau selon la revendication 1, dans lequel, lorsque le signal de contact est supprimé, la commande (600) arrête le fonctionnement de la partie de génération de haute fréquence (300).

3. Le dispositif de traitement de la peau selon la revendication 1, dans lequel la pointe d'électrode (200) est disposée sur une surface circonférentielle interne de la partie de corps principal (100) pour faire saillie vers l'intérieur.

4. Le dispositif de traitement de la peau selon la revendication 1, dans lequel la pointe d'électrode (200) comprend une pointe positive (210) et une pointe négative (220), et
la pointe positive (210) et la pointe négative (220) sont disposées sur une surface circonférentielle interne de la partie de corps principal (110) à des positions se faisant face.

5. Le dispositif de traitement de la peau selon la revendication 1, dans lequel le capteur de contact (500) est disposé à une position parallèle ou inférieure à la pointe d'électrode (200).

6. Le dispositif de traitement de la peau selon la revendication 1, dans lequel la commande (600), lorsqu'elle reçoit le second signal de contact, arrête le fonctionnement de la pompe d'aspiration (400), ou réduit la puissance d'aspiration de la pompe d'aspiration (400).

7. Le dispositif de traitement de la peau selon la revendication 1, comprenant en outre un capteur de température (700) disposé autour de la pointe d'électrode, dans lequel la commande (600) est en outre configurée pour fournir une notification ou commander le fonctionnement de la partie de génération de haute fréquence, en fonction d'une température détectée par le capteur de température (700).
